(19) Europäisches Patentamt
European Patent Office
Office européen des brevets

(11) EP 2 101 746 B1

(12) EUROPEAN PATENT SPECIFICATION

(45) Date of publication and mention
of the grant of the patent:
**11.12.2013 Bulletin 2013/50**

(51) Int Cl.:
*A61K 31/00* (2006.01)          *A61K 31/404* (2006.01)
*A61K 31/517* (2006.01)          *A61P 25/02* (2006.01)
*A61P 29/00* (2006.01)

(21) Application number: **07857560.2**

(22) Date of filing: **13.12.2007**

(86) International application number:
**PCT/EP2007/063913**

(87) International publication number:
**WO 2008/071779 (19.06.2008 Gazette 2008/25)**

(54) **V3 ANTAGONISTS FOR THE TREATMENT OF NEUROPATHIC PAIN**

V3-ANTAGONISTEN ZUR BEHANDLUNG VON NEUROPATHISCHEN SCHMERZEN

ANTAGONISTES DE V3 POUR LE TRAITEMENT D'UNE DOULEUR NEUROPATHIQUE

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **13.12.2006 EP 06126003**

(43) Date of publication of application:
**23.09.2009 Bulletin 2009/39**

(73) Proprietor: **Merck Sharp & Dohme B.V.
2031 BN Haarlem (NL)**

(72) Inventor: **NEUMANN, Inga
93040 Regensburg (DE)**

(74) Representative: **Jaap, David Robert
Merck & Co., Inc.
Hertford Road
Hoddesdon, Herts EN11 9BU (GB)**

(56) References cited:
**EP-A- 1 842 555          WO-A-2006/070742
WO-A-2006/095014          US-A1- 2003 114 683**

- **DRUMMOND P D: "The effect of noradrenaline, angiotensin II and vasopressin on blood flow and sensitivity to heat in capsaicin-treated skin." CLINICAL AUTONOMIC RESEARCH : OFFICIAL JOURNAL OF THE CLINICAL AUTONOMIC RESEARCH SOCIETY APR 1998, vol. 8, no. 2, April 1998 (1998-04), pages 87-93, XP002416511 ISSN: 0959-9851**
- **Mark S Wallace: "Pharmacologic Treatment of Neuropathic Pain", Curr Pain Headache Rep. 2001 Apr;5(2):138-50, 1 May 2001 (2001-05-01), XP055065692, Retrieved from the Internet: URL: http://rd.springer.com/content/pdf/10. 1007/s11916-001-0082-2.pdf [retrieved on 2013-06-06]**

**Description**

The present invention relates to a vasopressin type 3 ($V_3$ or $V_{1b}$) antagonist for use in the treatment of chronic pain which is neuropathic pain, wherein the $V_3$ antagonist is selected from a compound listed in claim 1.

Acute pain and chronic pain differ in their etiology, pathophysiology, diagnosis and treatment. Acute pain, which occurs following tissue injury, is self-limiting, serves as an alert to ongoing tissue damage and following tissue repair it will usually subside. There are minimal psychological symptoms associated with acute pain apart from mild anxiety. Acute pain is nociceptive in nature and occurs following chemical, mechanical and thermal stimulation of A-delta and C-polymodal pain receptors.

Chronic pain, on the other hand, serves no protective biological function. Rather than being the symptom of tissue damage it is a disease in its own right. Chronic pain is unrelenting and not self-limiting and can persist for years, perhaps decades after the initial injury. Chronic pain can be refractory to multiple treatment regimes. Psychological symptoms associated with chronic pain include chronic anxiety, fear, depression, sleeplessness and impairment of social interaction. Chronic non-malignant pain is predominantly neuropathic in nature and involves damage to either the peripheral or central nervous systems.

Acute pain and chronic pain are caused by different neuro-physiological processes and therefore tend to respond to different types of treatments. Acute pain can be somatic or visceral in nature. Somatic pain tends to be a well localised, constant pain and is described as sharp, aching, throbbing or gnawing. Visceral pain, on the other hand, tends to be vague in distribution, paroxysmal in nature and is usually described as deep, aching, squeezing or colicky in nature. Examples of acute pain include post-operative pain, pain associated with trauma and the pain of arthritis. Acute pain usually responds to treatment with opioids or non-steroidal anti-inflammatory drugs.

Chronic pain, in contrast to acute pain, is described as burning, electric, tingling and shooting in nature. It can be continuous or paroxysmal in presentation. The hallmarks of chronic pain are chronic allodynia and hyperalgesia. Allodynia is pain resulting from a stimulus that normally does not ellicit a painful response, such as a light touch.

Hyperalgesia is an increased sensitivity to normally painful stimuli. Primary hyperalgesia occurs immediately within the area of the injury. Secondary hyperalgesia occurs in the undamaged area surrounding the injury. Examples of chronic pain include complex regional pain syndromes, peripheral neuropathies, mechanical nerve injury and severe pain associated with diseases such as cancer, metabolic disease, neurotropic viral disease, neurotoxicity and multiple sclerosis. Chronic pain tends to be only partially responsive to treatment with opioid drugs.

Although opioids are cheap and effective, serious and potentially life-threatning side effects occur with their use, most notably respiratory depression and muscle rigidity. In addition the doses of opioids which can be administered are limited by nausea, emesis, constipation, pruritis and urinary retention, often resulting in patients electing to receive sub-optimal pain control rather than suffer these distressing side-effects. Furthermore, these side-effects often result in patients requiring extended hospitalisation. Opioids are highly addictive and are scheduled drugs in many territories.

Efforts continue therefore to find new treatments for pain management, in particular treatment of neuropathic pain. Moreover treatments of pain management are needed which are safe as well as effective.

The present invention provides a vasopressin type 3 ($V_3$ or $V_{1b}$) antagonist for use in the treatment of chronic pain which is neuropathic pain, wherein the $V_3$ antagonist is selected from the compounds listed in claim 1.

The actions of arginine vasopressin (AVP) at the pituitary corticotrope are mediated by the vasopressin type 3 ($V_3$ or $V_{1b}$) receptor, which is known and has been cloned (human receptor: Sugimoto et al., J. Biol. Chem., 1994, 269, 27088-27092). In addition to the $V_3$ receptor, vasopressin also activates other vasopressin receptors, i.e., the $V_{1a}$ receptor, found in the brain and in liver and vascular tissue and the $V_2$ receptor, predominantly found in kidney tissue. Interaction at these receptors mediates the pressor and antidiuretic actions of AVP.

$V_3$ receptor antagonists and their uses are known in the art. For example, US 2003/114683 relates to certain 1,3-dihydro-2H-indol-2-one derivatives, for example SSR 149415, indicated to be useful in the treatment of CNS disorders such as stress, anxiety, depression, obsessive compulsive disorder, panic attacks, psychotic states and memory troubles. More recently WO 2006/095014 has disclosed a series of 2-(4-oxo-4H-quinazolin-3-yl)acetamide derivatives also indicated to be useful for the treatment or prevention of disorders or diseases influenced by modulation of the activity of the HPA axis, such as depression.

Database WPI, Derwent Publications Ltd., Class 065, page 1, AN 2006-5029261 XP002416515 (WO 2006/070742) indicates that SSR 149415 inhibits the tolerance development to the analgesic activity of morphine. There is no suggestion, however, of the possibility that SSR 149415 would be effective, in its own right, in the treatment of neuropathic pain. Furthermore, there is no suggestion that SSR 149415 would be effective in the treatment of neuropathic pain whilst showing little or no effect in the treatment of acute pain as has now been found. US 2003114683 indicates that the $V_3$ antagonists disclosed therein are useful in a range of disorders including the treatment of pain in general and pain-perception disorders although no supporting experimental data is provided. However likewise, there is no suggestion in US 2003114683 of the possibility that $V_3$ antagonists might be especially effective in the treatment of neuripathic pain whilst showing little or no effect against acute pain as has now been found.

MS Wallace ("Pharmacologic Treatment of Neuropathic Pain", Curr Pain Headache Rep. 2001 Apr;5(2):138-50. Review) gives an overview on the pharmacological treatment of neuropathic pain. Neuropathic pain appears to be a specific pain state that needs to be treated differently as common pain since analgesics such as NSAIDs appear to be ineffective. A summary on the efficacy of current available agents, such as opiates, NMDA antagonists and antidepressants is given. V3 antagonists are not mentioned.

Also disclosed are all stereoisomeric forms of the $V_3$ antagonists according to claim 1 as described herein resulting, for example, because of configurational or geometrical isomerism. Such stereoisomeric forms are enantiomers, diastereoisomers, *cis* and *trans* isomers etc. For example, in the case where R[1] is 2-methylbutyl the compound exists as a pair of enantiomers. In the case where R[4] comprises an alkene fragment, both (Z) and (E) stereoisomeric forms of the compound are possible. In the case of the individual enantiomers of compounds of formula I or salts or solvates thereof, the present invention includes use of the aforementioned stereoisomers substantially free, *i.e.,* associated with less than 5%, preferably less than 2% and in particular less than 1% of the other enantiomer. Mixtures of stereoisomers in any proportion, for example a racemic mixture comprising substantially equal amounts of two enantiomers are also disclosed.

The present invention relates to a $V_3$ receptor antagonist for use in the treatment of neuropathic pain, wherein said $V_3$ receptor antagonist is selected from:

(2S,4R)-1-[5-chloro-1-(2,4-dimethoxyphenyl)sulfonyl]-3-(2-methoxyphenyl)-2-oxo-2,3-dihydro-1*H*-indol-3-yl]-4-hydroxy-*N,N*-dimethyl-2-pyrrolidine carboxamide;

2-[2-(3-Chloro-4-fluorophenyl)-4-oxo-6-(3-piperidin-1-ylpropoxy)-4*H*-quinazolin-3-yl]-*N*-isopropylacetamide;
*N*-Isopropyl-2-[2-(3-methoxyphenyl)-4-oxo-6-(3-piperidin-1-ylpropoxy)-4*H*-quinazolin-3-yl]acetamide;
2-[2-(4-Fluoro-3-methoxyphenyl)-4-oxo-6-(3-piperidin-1-ylpropoxy)-4*H*-quinazolin-3-yl]-*N*-isopropylacetamide;
2-[2-(3-Chlorophenyl)-6-[3-(4-hydroxypiperidin-1-yl)propoxy]-4-oxo-4*H*-quinazolin-3-yl]-*N*-isopropylacetamide;
2-[2-(3-Chlorophenyl)-4-oxo-6-(3-piperidin-1-ylpropoxy)-4*H*-quinazolin-3-yl]-*N* isopropylacetamide;
(S)-(+)-2-[2-(3-Chlorophenyl)-6-(2-methyl-3-pyrrolidin-1-ylpropoxy)-4-oxo-4*H*-quinazolin-3-yl]-*N*-isopropylacetamide;
2-[6-(5-Dimethylaminomethyl-2-fluorophenyl)-2-(3-methoxyphenyl)-4-oxo-4*H*-quinazolin-3-yl]-*N*-isopropylacetamide;
2-[6-(3-Dimethylaminomethylphenyl)-2-(3-methoxyphenyl)-4-oxo-4H-quinazolin-3-yl]-*N*-isopropylacetamide;
*N-tert*-Butyl-2-[2-(3-chlorophenyl)-4-oxo-6-(3-pyrrolidin-1-ylpropoxy)-4*H*-quinazolin-3-yl] acetamide and
2-[6-(3-Dimethylaminomethylphenyl)-2-(4-fluoro-3-methoxyphenyl)-4-oxo-4*H*-quinazolin-3-yl]-*N*-isopropylacetamide

or a pharmaceutically acceptable salt or solvate thereof.

Pharmaceutical compositions for the use as claimed and described herein were prepared in accordance with standard techniques in the art of pharmaceutical sciences. The compounds can be used for humans in a dosage of 0.001-50 mg per kg body weight, preferably in a dosage of 0.01-20 mg per kg body weight, whereby the optimum dosage can be determined according to factors such as route of administration, desired duration of action, type of formulation (extended release versus immediate release), type of patient, type of compound required, efficacy of the compound and other physical characteristics of the recipient of the treatment, such co-morbidity of other diseases, liver metabolism capacity, etc.

$V_3$ receptor antagonism and methods how to determine such a biological effect can be determined according to known techniques in the biochemistry of G-protein coupled receptors. A specific method is described in the example below, on which basis a criterion pKi value of at least 6.0, or preferably 6.5, or even better 7.0 can be derived for clarity of the meaning of the term $V_3$ receptor antagonist. Further $V_3$ receptor antagonists can be identified in this way.

For diagnostic criteria for chronic pain reference is made to the DSM IV revised edition.

The invention is further illustrated by the following examples.

Example 1 - Determination of $V_3$ receptor Antagonism

Chinese Hamster Ovary (CHO) cells stably expressing the human $V_3$ receptor were incubated to equilibrium with the test compound (at a final assay concentration of $10^{-10}$ mol.L$^{-1}$ to $10^{-5}$ mol.L$^{-1}$) and [$^3$H]AVP (at a final assay concentration of $2.5 \times 10^{-9}$ mol.L$^{-1}$). Throughout the concentration of dimethylsulphoxide (DMSO) did not exceed 0.1% (v/v). After washing with ice-cold phosphate buffered saline (PBS), scintillation fluid was added and the plates counted on a Topcount NXT apparatus.

A sigmoidal dose response curve (non-linear regression, variable slope) was plotted as concentration of test compound mol.L$^{-1}$) against percentage specific binding of [$^3$H]AVP and a $K_i$ value was calculated. Each determination was carried out in triplicate and repeated on at least 3 separate occasions

The ability of compounds of the invention to act as $V_3$ antagonists in a physiologically relevant system was determined by measuring their ability to block the release of adrenocorticotropic hormone (ACTH) from anterior pituitary corticotrophs in response to treatment with arginine vasopressin (AVP).

Anterior pituitary corticotrophs were prepared from adult female Sprague-Dawley rats and seeded into 48 well plates. The cells were cultured for 4 days prior to exposure to compound. Test compounds were prepared at $10^{-5}$ mol.$L^{-1}$ in 100% DMSO. Cells were exposed to a dose response of test compounds for 20 minutes ($10^{-8}$ mol.$L^{-1}$ - $10^{-5}$ mol.$L^{-1}$). The final concentration of DMSO in the assay was kept constant at 0.3%. The cells were then exposed to $3 \times 10^{-9}$ mol.$L^{-1}$ AVP for 120 minutes. Supernatants were harvested and stored at -20°C. ACTH levels were subsequently measured by ELISA following the manufacturer's instructions (Immunodiagnostic systems, UK (Cat No. DX-SDX018)). Each treatment was carried out in quadruplicate and a mean value obtained for the amount of ACTH released. The degree of antagonism was then calculated as a percentage of the amount of ACTH released by agonist alone after adjustment for basal levels of ACTH. A $pIC_{50}$ was calculated by fitting a Sigmoidal dose response (variable slope) curve with a non-linear (fit) to the data using the software package GraphPad prism. Each determination was repeated on at least 3 separate occasions

Example 2 - Evaluation of Selective $V_3$ receptor antagonists in a rat (Chung) model of neuropathic pain.

In this model, mechanical allodynia is induced by tight ligation of the left L5 spinal nerve. This assay has been employed successfully to demonstrate anti-allodynic effects of anticonvulsants (gabapentin), antidepressants (duloxetine) and opioid analgesics (morphine) which are used clinically in the treatment of neuropathic pain.

Male Wistar rats (150-175 g body weight at time of surgery) were employed in the study. Rats were placed on an elevated (~40cm) mesh floor in perspex boxes and the rats' withdrawal threshold to a mechanical stimulus (calibrated von Frey filaments) was measured using filaments of increasing force (2.6-167 mN). The von Frey filaments were applied to the plantar surface of the paw and the threshold response was determined using the up and down method. A positive response was noted if the paw was sharply withdrawn. A cut-off of 15g was selected as the upper limit for testing. Following baseline measurements each animal was anaesthetised and the L5 spinal nerve tightly ligated. The animals were allowed to recover from the surgery for a period of at least three days. On the day of drug administration the paw withdrawal thresholds were re-measured (0 min). Immediately after this reading, the rats were dosed orally with vehicle or test compound. Readings were then made at intervals of 30, 60, 120, 180 and 240 min after compound administration.

Data were expressed as mean ± s.e.m. The time of maximum effect for each animal in the top dose group was determined and these values averaged to calculate the mean time of maximum effect. For analytical purposes the time of maximum effect, $t_{max}$ was defined as the time point closest to this averaged value. Data at $t_{max}$ were compared between groups using the Kruskal-Wallis one-way analysis of variance, a non-parametric statistical test. Each of the treatment groups were then compared against the vehicle group, using the non-parametric Dunn's test. The $ED_{50}$ (dose at which allodynia is reversed by 50%) value was also calculated at $t_{max}$ using non linear regression (sigmoidal dose response; variable slope) with constants of 0 and 15g (cut-off) for the bottom and top, respectively (XLFit software).

Three $V_3$ antagonists were used in the experiment:

Compound 1, *i.e.*, 2-[6-(3-Dimethylaminomethylphenyl)-2-(4-fluoro-3-methoxyphenyl)-4-oxo-4*H*-quinazolin-3-yl]-*N*-isopropylacetamide;

Compound 2, *i.e.,* 2-[3-(3-chlorophenyl)-4-oxo-6-(3-piperidin-1-ylpropoxy)-4*H*-quinazolin-3-yl]-*N*-isopropylacetamide and

Compound 3, *i.e.,* (2*S*,4*R*)-1-[5-chloro-1-(2,4-dimethoxyphenyl)sulfonyl]-3-(2-methoxyphenyl)-2-oxo-2,3-dihydro-1*H*-indol-3-yl]-4-hydroxy-*N,N*-dimethyl-2-pyrrolidine carboxamide *i.e.,* SSR149415;

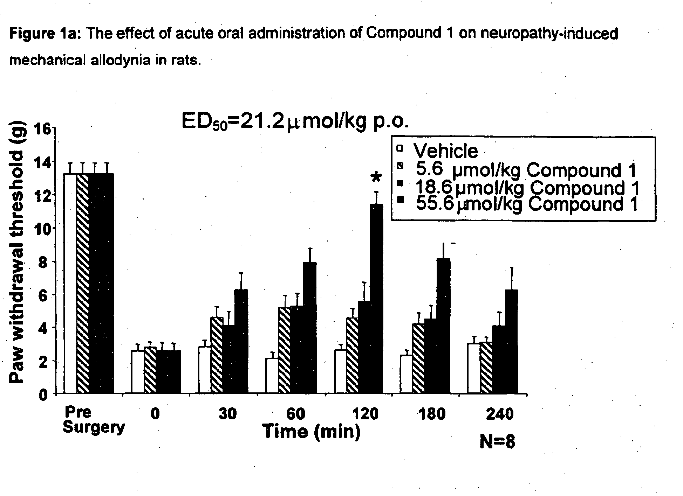

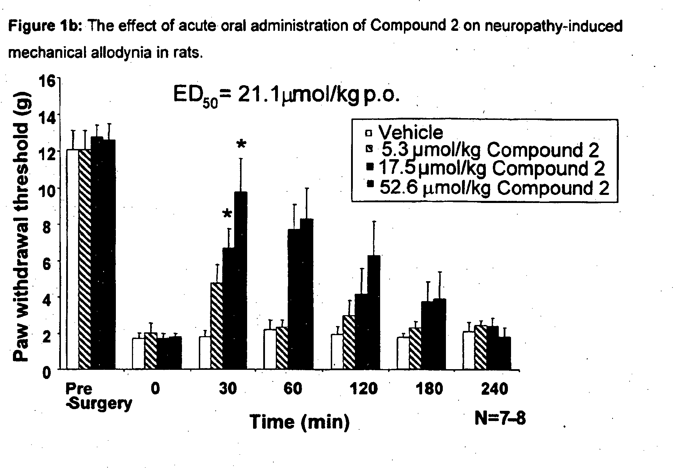

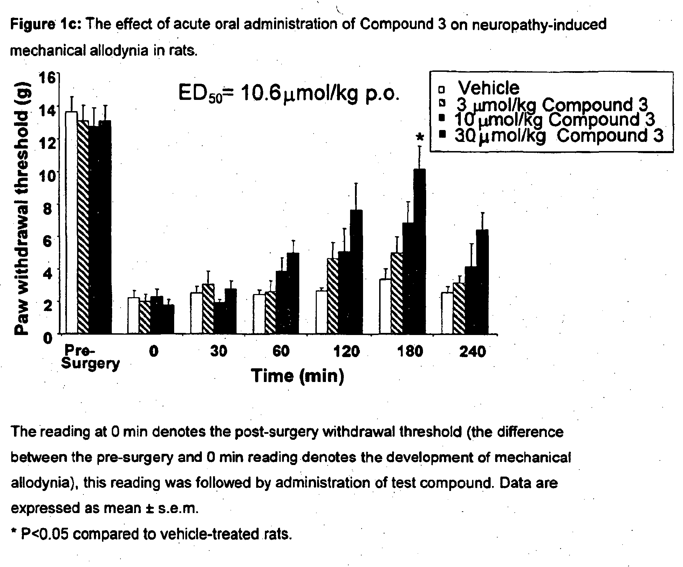

Oral administration of these compounds, compound 1, (5.6, 18.6 or 55.6 μmol/kg), compound 2, (5.3, 17.3 or 52.6 μmol/kg) or compound 3, (3, 10, or 30 μmol/kg) in rats reversed mechanical allodynia in a dose-dependent fashion (Table 1; Figure 1a, b, c, respectively). The peak anti-allodynic effect for compound 1 was observed at 120 min (Figure 1 a), for compound 2 at 30 min (Figure 1 b) and for compound 3 at 180 min (Figure 1 c) post drug administration. The calculated $ED_{50}$ values at peak effect were 21.2 μmol/kg p.o. for compound 1, 21.0 μmol/kg for compound 2 and 10.6 μmol/kg for compound 3.

These data demonstrate that $V_3$ receptor antagonists possess potent oral anti-allodynic activity in a rat model of neuropathic pain, a form of chronic pain.

**Table 1:** Effect of 3 V$_3$ antagonists on mechanical allodynia induced by spinal nerve ligation in rats. Dose groups and number of animals per group. *p $\leq$ 0.01 ** p $\leq$ 0.001, Dunns test comparing vehicle-treated and compound-treated animals.

| Compound | Route | Dose (μmol/kg) | Number of animals tested | Withdrawal threshold (g) at peak effect |
|---|---|---|---|---|
| Vehicle | p.o. | 1ml.kg$^{-1}$ | 8 | 2.6 $\pm$ 0.3 |
| Compound 1 | p.o. | 5.6 | 8 | 4.6 $\pm$ 0.6 |
| Compound 1 | p.o. | 18.6 | 8 | 5.6 $\pm$ 1.1 |
| Compound 1 | p.o. | 55.6 | 8 | 11.4 $\pm$ 0.7** |
| | | | | |
| Vehicle | p.o. | 1ml.kg$^{-1}$ | 8 | 1.8 $\pm$ 0.3 |
| Compound 2 | p.o. | 5.3 | 8 | 4.8 $\pm$ 1.0 |
| Compound 2 | p.o. | 17.3 | 8 | 6.7 $\pm$ 1.1* |
| Compound 2 | p.o. | 52.6 | 7 | 9.8 $\pm$ 1.8** |
| | | | | |
| Vehicle | p.o. | 1ml.kg-1 | 7 | 3.4 $\pm$ 0.7 |
| Compound 3 | p.o. | 3 | 7 | 5.0 $\pm$ 1.0 |
| Compound 3 | p.o. | 10 | 7 | 6.9 $\pm$ 1.3 |
| Compound 3 | p.o. | 30 | 7 | 10.1 $\pm$ 1.4** |

Reference Example 3 Evaluation of the V$_3$ receptor antagonist Compound 2 on mechanical hyperalgesia in a rat model of inflammatory pain.

In this model, inflammation is induced by subcutaneous injection of complete Freund's adjuvant (CFA) into the hind paw. The associated mechanical hyperalgesia is quantified by measuring the reduction in paw withdrawal threshold (PWT) to a mechanical compression of the paw. This assay has been employed successfully to demonstrate anti-hyperalgesic effects of non-steroidal anti-inflammatory drugs (indomethacin) and cyclooxygenase-2 inhibitors (celecoxib) which are used clinically in the treatment of chronic inflammatory pain.

Experiments were in male Wistar rats weighing (130-180 g). In brief, the rats' paw withdrawal threshold (PWT) to a mechanical compression of the hind paw was measured (baseline reading) using a Randall-Sellito apparatus (Ugo Basile). A cut-off of 200 g was employed to minimise tissue damage to the paw. The animals were then lightly anaesthetised with isoflurane (1-3 %) and CFA (0.1 ml.paw$^{-1}$) injected subcutaneously (s.c.) into the plantar surface of the left hind paw. The animals were then returned to their home cage and left for the inflammation to develop.

Seven days after CFA injection, PWT's were re-measured (0 min) and immediately after this reading, rats were dosed orally with either vehicle or compound 2 (3-30 mg/kg). Readings were then made at 30 min and 2 h post drug administration.

Data were plotted as mean $\pm$ s.e.m. and compared between groups using the Kruskal-Wallis one-way analysis of variance, a non-parametric statistical test. If statistical significance (P< 0.05) was observed with this test, the vehicle group and each of the treatment groups were compared using the non-parametric Dunn's test. The percent attenuation of mechanical hyperalgesia is calculated at time of peak effect (t$_{max}$).

$$\% \text{ attenuation of hyperalgesia} = \frac{(\text{Post compound PWT} - \text{post CFA PWT})}{(\text{Baseline PWT} - \text{post CFA PWT})} \times 100$$

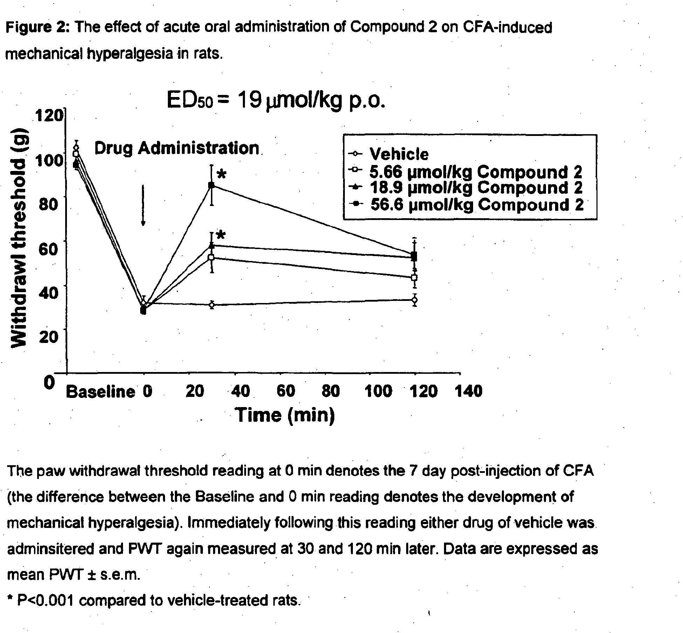

Oral administration of Compound 2 (5.7, 18.9 or 56.6 μmol/kg) reversed mechanical hyperalgesia induced by CFA in a dose-dependent fashion (Table 2; Figure 2). The peak anti-algesic effect was observed at 30 min post drug administration (Figure 2). The calculated ED$_{50}$ value at peak effect was 19.2 μmol/kg p.o.

These data demonstrate that the V$_3$ receptor antagonist Compound 2 possesses potent oral anti-algesic activity in a rat model of chronic inflammatory pain.

**Table 2:** Effect of Compound 2 on mechanical hyperalgesia induced by complete Freund's adjuvant administered 7 days previously in rats. Dose groups and number of animals per group. * p $\leq$ 0.01 ** p $\leq$ 0.001, Dunns test comparing vehicle-treated and compound-treated animals.

| Compound | Route | Dose ($\mu$mol/kg) | Number of animals tested | % Attenuation of hyperalgesia (peak effect) |
|---|---|---|---|---|
| Vehicle | p.o. | 3ml.kg$^{-1}$ | 8 | -2.2 $\pm$ 2.6 |
| Compound 2 | p.o. | 5.7 | 8 | 31.5 $\pm$ 9.4 |
| Compound 2 | p.o. | 18.9 | 8 | 43.1 $\pm$ 8.6** |
| Compound 2 | p.o. | 56.6 | 8 | 84.4 $\pm$ 12.0** |

Reference Example 4 - Evaluation of the V$_3$ Receptor Antagonists In Acute Thermal Pain Using the Mouse tail Flick Assay.

The tail flick test is used to evaluate the antinociceptive activity of test compounds in mice. Animals are placed on the tail flick apparatus and their tails exposed to a focused beam of radiant heat. The mice react to the noxious thermal stimulus by flicking their tail away from the heat source. The time taken for these responses to occur is measured (latency; s). This assay has been employed successfully to demonstrate antinociceptive effects of opioids (morphine, fentanyl) and adrenergic agonists (clonidine) which are used clinically for the treatment of moderate pain.

Experiments were performed using male ICR mice weighing (25-32g). In brief, animals were held in place on the tail flick apparatus (Ugo Basile, Italy) by means of a perspex restrainer and their tail was positioned over the heat source. The latency to flick the tail from the radiant heat source focussed 2.5 cm from the tip was determined before (baseline readings) and at regular intervals after administration of the vehicle or test compounds (compound 1, 5.6-55.6 $\mu$mol/kg p.o.; compound 2, 5.3-52.6 $\mu$mol/kg p.o.). A 12 seconds cut-off was employed to prevent tissue damage.

Data are expressed as mean $\pm$ s.e.m. and compared between groups using the Kruskal-Wallis one-way analysis of variance, a non-parametric statistical test. If statistical significance (P < 0.05) is observed with this test, the vehicle group and each of the treatment groups is compared using the non-parametric Dunn's test.

Oral administration of compound 1, (5.6, 18.6 or 55.6 $\mu$mol/kg) or compound 2, (5.3, 17.3 or 52.6 $\mu$mol/kg) to mice had no effect on tail flick latencies when measured 20, 40 and 60 min post compound administration (Table 3).

These data demonstrate that the V$_3$ receptor antagonists do not posses antinociceptive activity in a mouse model of acute thermal pain.

**Table 3:** Lack of effect of Compound 1 and 2 on tail flick latency in mice. Dose groups and number of animals per group. No significant difference was observed using Kruskal-Wallis analysis of variance at any of the time points.

| Compound | Route | Dose ($\mu$mol/kg) | Number of animals tested | Tail Flick Latency (s) | | |
|---|---|---|---|---|---|---|
| | | | | 20 min post dose | 40 min post dose | 60 min post dose |
| Vehicle | p.o. | 1ml.kg$^{-1}$ | 8 | 3.4 $\pm$ 0.3 | 3.2 $\pm$ 0.2 | 3.3 $\pm$ 0.1 |
| Compound 1 | p.o. | 5.6 | 8 | 3.5 $\pm$ 0.4 | 3.4 $\pm$ 0.2 | 3.5 $\pm$ 0.2 |
| Compound 1 | p.o. | 18.6 | 8 | 3.4 $\pm$ 0.3 | 3.5 $\pm$ 0.2 | 3.1 $\pm$ 0.2 |
| Compound 1 | p.o. | 55.6 | 8 | 3.3 $\pm$ 0.3 | 3.5 $\pm$ 0.3 | 3.3 $\pm$ 0.2 |
| | | | | | | |
| Vehicle | p.o. | 1ml.kg$^{-1}$ | 8 | 3.2 $\pm$ 0.1 | 3.3 $\pm$ 0.1 | 3.3 $\pm$ 0.1 |
| Compound 2 | p.o. | 5.3 | 8 | 3.4 $\pm$ 0.2 | 3.7 $\pm$ 0.2 | 3.6 $\pm$ 0.1 |
| Compound 2 | p.o. | $\pm$ 17.3 | 8 | 3.6 $\pm$ 0.1 | 3.6 $\pm$ 0.1 | 3.1 $\pm$ 0.1 |
| Compound 2 | p.o. | 52.6 | 8 | 3.5 $\pm$ 0.2 | 3.5 $\pm$ 0.2 | 3.2 $\pm$ 0.2 |

**Claims**

1. A V$_3$ receptor antagonist for use in the treatment of chronic pain which is neuropathic pain, which is selected from:

(2S,4R)-1-[5-chloro-1-(2,4-dimethoxyphenyl)sulfonyl]-3-(2-methoxyphenyl)-2-oxo-2,3-dihydro-*1H*-indol-3-yl]-4-hydroxy-*N,N*-dimethyl-2-pyrrolidine carboxamide;
2-[2-(3-Chloro-4-fluorophenyl)-4-oxo-6-(3-piperidin-1-ylpropoxy)-4*H*-quinazolin-3-yl]-*N* isopropylacetamide;
*N*-Isopropyl-2-[2-(3-methoxyphenyl)-4-oxo-6-(3-piperidin-1-ylpropoxy)-4*H*-quinazolin-3-yl]acetamide;
2-[2-(4-Fluoro-3-methoxyphenyl)-4-oxo-6-(3-piperidin-1-ylpropoxy)-4*H*-quinazolin-3-yl]-*N*-isopropylacetamide;
2-[2-(3-Chlorophenyl)-6-[3-(4-hydroxypiperidin-1-yl)propoxy]-4-oxo-4*H*-quinazolin-3-yl]-*N*-isopropylacetamide;
2-[2-(3-Chlorophenyl)-4-oxo-6-(3-piperidin-1-ylpropoxy)-4*H*-quinazolin-3-yl]-*N* isopropylacetamide;
(S)-(+)-2-[2-(3-Chlorophenyl)-6-(2-methyl-3-pyrrolidin-1-ylpropoxy)-4-oxo-4*H*-quinazolin-3-yl]-*N*-isopropylacetamide;
2-[6-(5-Dimethylaminomethyl-2-fluorophenyl)-2-(3-methoxyphenyl)-4-oxo-4*H*-quinazolin-3-yl]-*N*-isopropylacetamide;
2-[6-(3-Dimethylaminomethylphenyl)-2-(4,    fluoro-3-methoxyphenyl)-4-oxo-4H-quinazolin-3-yl]-*N*-isopropylacetamide;
*N-tert*-Butyl-2-[2-(3-chlorophenyl)-4-oxo-6-(3-pyrrolidin-1-ylpropoxy)-4*H*-quinazolin-3-yl] acetamide and
2-[6-(3-Dimethylaminomethylphenyl)-2-(3-methoxyphenyl)-4-oxo-4*H*-quinazolin-3-yl]-*N*-isopropylacetamide

or a pharmaceutically acceptable salt or solvate thereof.

**Patentansprüche**

1. Ein V$_3$-Rezeptor-Antagonist zur Verwendung bei der Behandlung chronischer Schmerzen, die neuropathische Schmerzen sind, ausgewählt aus:

(2S,4R)-1-[5-Chlor-1-(2,4-dimethoxyphenyl)sulfonyl]-3-(2-methoxyphenyl)-2-oxo-2,3-dihydro-1*H*-indol-3-yl]-4-hydroxy-*N,N*-dimethyl-2-pyrrolidincarboxamid,
2-[2-(3-Chlor-4-fluorphenyl)-4-oxo-6-(3-piperidin-1-ylpropoxy)-4*H*-chinazolin-3-yl]-N-isopropylacetamid,
*N*-Isopropyl-2-[2-(3-methoxyphenyl)-4-oxo-6-(3-piperidin-1-ylpropoxy)-4*H*-chinazolin-3-yl]acetamid,
2-[2-(4-Fluor-3-methoxyphenyl)-4-oxo-6-(3-piperidin-1-ylpropoxy)-4*H*-chinazolin-3-yl]-*N*-isopropylacetamid,
2-[2-(3-Chlorphenyl)-6-[3-(4-hydroxypiperidin-1-yl)propoxy]-4-oxo-4*H*-chinazolin-3-yl]-*N*-isopropylacetamid,
2-[2-(3-Chlorphenyl)-4-oxo-6-(3-piperidin-1-ylpropoxy)-4*H*-chinazolin-3-yl]-*N*-isopropylacetamid,
(S)-(+)-2-[2-(3-Chlorphenyl)-6-(2-methyl-3-pyrrolidin-1-ylpropoxy)-4-oxo-4*H*-chinazolin-3-yl]-*N*-isopropylacetamid,
2-[6-(5-Dimethylaminomethyl-2-fluorphenyl)-2-(3-methoxyphenyl)-4-oxo-4*H*-chinazolin-3-yl]-*N*-isopropylacetamid,
2-[6-(3-Dimethylaminomethylphenyl)-2-(4-fluor-3-methoxyphenyl)-4-oxo-4*H*-chinazolin-3-yl]-*N*-isopropylacetamid,
*N-tert*-Butyl-2-[2-(3-chlorphenyl)-4-oxo-6-(3-pyrrolidin-1-ylpropoxy)-4*H*-chinazolin-3-yl]acetamid und
2-[6-(3-Dimethylaminomethylphenyl)-2-(3-methoxyphenyl)-4-oxo-4*H*-chinazolin-3-yl]-*N*-isopropylacetamid

oder einem pharmazeutisch annehmbaren Salz oder Solvat davon.

**Revendications**

1. Antagoniste du récepteur V$_3$ à utiliser dans le traitement d'une douleur chronique qui est une douleur neuropathique, lequel est sélectionné parmi:

(2S,4R)-1-[5-chloro-1-(2,4-diméthoxyphényl)sulfonyl]-3-(2-méthoxyphényl)-2-oxo-2,3-dihydro-1*H*-indol-3-yl]-4-hydroxy-*N,N*-diméthyl-2-pyrolidine carboxamide;
2-[2-(3-chloro-4-fluorophényl)-4-oxo-6-(3-pipéridin-1-ylpropoxy)-4*H*-quinazolin-3-yl]-*N*-isopropylacétamide;
*N*-isopropyl-2-[2-(3-méthoxyphényl)-4-oxo-6-(3-pipéridin-1-ylpropoxy)-4*H*-quinazolin-3-yl]acétamide;
2-[2-(4-fluoro-3-méthoxyphényl)-4-oxo-6-(3-pipéridin-1-ylpropoxy)-4*H*-quinazolin-3-yl]-*N*-isopropylacétamide;

2-[2-(3-chlorophényl)-6-[3-(4-hydroxypipéridin-1-yl)propoxy]-4-oxo-4*H*-quinazolin-3-yl]-*N*-isopropylacétamide;
2-[2-(3-chlorophényl)-4-oxo-6-(3-pipéridin-1-ylpropoxy)-4*H*-quinazolin-3-yl]-*N*-isopropylacétamide;
(S)-(+)-2-[2-(3-chlorophényl)-6-(2-méthyl-3-pyrrolidin-1-ylpropoxy)-4-oxo-4*H*-quinazolin-3-yl]-*N*-isopropylacétamide;
2-[6-(5-diméthylaminométhyl-2-fluorophényl)-2-(3-méthoxyphényl)-4-oxo-4*H*-quinazolin-3-yl]-*N*-isopropylacétamide;
2-[6-(3-diméthylaminométhylphényl)-2-(4,fluoro-3-méthoxyphényl)-4-oxo-4*H*-quinazolin-3-yl]-*N*-isopropylacétamide;
*N-tert*-butyl-2-[2-(3-chlorophényl)-4-oxo-6-(3pyrrolidin-1-ylpropoxy)-4*H*-quinazolin-3-yl]acétamide; et
2-[6-(3-diméthylaminométhylphényl)-2-(3-méthoxyphényl)-4-oxo-4*H*-quinazolin-3-yl]-*N*-isopropylacétamide

ou un sel ou un solvate pharmaceutiquement acceptable de ceux-ci.

**Figure 1a:** The effect of acute oral administration of Compound 1 on neuropathy-induced mechanical allodynia in rats.

$ED_{50} = 21.2 \mu mol/kg$ p.o.

Paw withdrawal threshold (g)

Legend:
- Vehicle
- 5.6 μmol/kg Compound 1
- 18.6 μmol/kg Compound 1
- 55.6 μmol/kg Compound 1

Time (min): Pre Surgery, 0, 30, 60, 120, 180, 240

N=8

**Figure 1b:** The effect of acute oral administration of Compound 2 on neuropathy-induced mechanical allodynia in rats.

$ED_{50} = 21.1 \mu mol/kg\ p.o.$

□ Vehicle
▨ 5.3 μmol/kg Compound 2
■ 17.5 μmol/kg Compound 2
■ 52.6 μmol/kg Compound 2

Paw withdrawal threshold (g)

Time (min)

N=7-8

**Figure 1c:** The effect of acute oral administration of Compound 3 on neuropathy-induced mechanical allodynia in rats.

The reading at 0 min denotes the post-surgery withdrawal threshold (the difference between the pre-surgery and 0 min reading denotes the development of mechanical allodynia), this reading was followed by administration of test compound. Data are expressed as mean ± s.e.m.

\* $P<0.05$ compared to vehicle-treated rats.

Figure 2: The effect of acute oral administration of Compound 2 on CFA-induced mechanical hyperalgesia in rats.

The paw withdrawal threshold reading at 0 min denotes the 7 day post-injection of CFA (the difference between the Baseline and 0 min reading denotes the development of mechanical hyperalgesia). Immediately following this reading either drug of vehicle was adminsitered and PWT again measured at 30 and 120 min later. Data are expressed as mean PWT ± s.e.m.

* P<0.001 compared to vehicle-treated rats.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2003114683 A
- WO 2006095014 A

- WO 2006070742 A

**Non-patent literature cited in the description**

- **SUGIMOTO et al.** *J. Biol. Chem.,* 1994, vol. 269, 27088-27092

- **MS WALLACE.** Pharmacologic Treatment of Neuropathic Pain. *Curr Pain Headache Rep.,* April 2001, vol. 5 (2), 138-50